# EUROPÄISCHE PATENTSCHRIFT

(11) **EP 0 536 175 B1**
(45) Veröffentlichungstag und Bekanntmachung des Hinweises auf die Patenterteilung: **20.09.1995**
(21) Anmeldenummer: 91910611.2
(22) Anmeldetag: 08.06.1991
(51) Int. Cl.: C07C 205/12, C07C 201/12

(54) **VERFAHREN ZUR HERSTELLUNG VON CHLORFLUORNITROBENZOLEN UND DIFLUORNITROBENZOLEN**
PROCESS FOR PRODUCING CHLOROFLUORONITRO BENZENES AND DIFLUORONITRO BENZENES
PROCEDE DE PRODUCTION DE CHLOROFLUORONITROBENZENES ET DE DIFLUORONITROBENZENES

(30) Priorität: 25.06.1990 DE 4020130; 31.08.1990 DE 4027591
(43) Veröffentlichungstag der Anmeldung: 14.04.1993
(73) Patentinhaber: HOECHST AKTIENGESELLSCHAFT, 65926 Frankfurt am Main (DE)
(72) Erfinder: PAPENFUHS, Theodor, D-6000 Frankfurt am Main (DE); KANSCHIK-CONRADSEN, Andreas, D-6084 Gernsheim/Rhein (DE); PRESSLER, Wilfried, D-6233 Kelkheim (DE)
(86) Internationale Anmeldenummer: EP9101079
(87) Internationale Veröffentlichungsnummer: WO9200270

(56) Entgegenhaltungen:
- DE-A- 2 938 939
- US-A- 4 642 399
- Chemical Abstracts, Band 111, Nr. 7, 14. August 1989, (Columbus, Ohio, US),siehe Seite 711, Zusammenfassung 57247a, & JP-A-01 13 037 (IHARA CHEMICAL INDUSTRY CO., LTD) 17. Januar 1989

## Beschreibung

Gegenstand der vorliegenden Erfindung ist ein Verfahren zur Herstellung von Chlorfluornitrobenzolen und Difluornitrobenzolen in hohen Ausbeuten ohne zusätzliche Reinigungsoperationen durch Umsetzung eines Überschusses der entsprechenden Dichlornitrobenzole mit Alkalimetallfluoriden eines gewissen Wassergehalts in Gegenwart von Katalysatoren und in Abwesenheit eines Lösungsmittels.

Chlorfluornitrobenzole und Difluornitrobenzole sind wichtige Zwischenprodukte für die Herstellung von Pharmazeutika und Pflanzenschutzmitteln.

Die US-PS 4 164 517 beschreibt die Umsetzung von 3,4- und 2,4-Dichlornitrobenzol zu Chlorfluor- bzw. Difluornitrobenzolen mit vorgetrocknetem Kaliumfluorid in Sulfolan bei Temperaturen oberhalb von 200°C. Dabei läßt sich die Ausbeute mit zunehmendem Anteil an Sulfolan erhöhen. So wird ausgeführt, daß bei der Umsetzung von 2,4-Dichlornitrobenzol ohne Sulfolan selbst nach einer Reaktionszeit von 30 h bei 240°C nur 20 % umgesetzt werden.

In der US-A 42 87 374 wird ein Verfahren zur Herstellung von Monofluornitrobenzolen aus insbesondere Monochlornitrobenzolen mit fein gepulvertem Kaliumfluorid in der Schmelze bei vorzugsweise 140 bis 150°C unter Zusatz von Tetraalkyl- oder Aralkylammoniumsalzen als Katalysatoren beschrieben. Dabei betragen die Reaktionszeiten gemäß den Beispielen 17 bis 28 h. Die Nachteile dieses Verfahrens sind, außer in der schlechten Raum-Zeit-Ausbeute, darin zu sehen, daß das vorzugsweise verwendete Kaliumfluorid einen Wassergehalt von weniger als 0,2 Gew.-% enthält, was eine Vorbehandlung des Kaliumfluorids erforderlich macht.

Es bestand daher ein erhebliches Interesse für ein technisch günstigeres Verfahren zur Herstellung von Chlorfluornitrobenzolen und Difluornitrobenzolen.

Es wurde nun Überraschenderweise gefunden, daß man Chlorfluornitrobenzole und Difluornitrobenzole in hohen Ausbeuten herstellen kann, indem man Dichlornitrobenzol im molaren Überschuß mit einem Alkalimetallfluorid mit einem Gesamtwassergehalt von etwa 0,2 bis etwa 2,5 Gew.-% in Gegenwart eines quartären Ammonium- und/oder Phosphoniumsalzes, Kronenäthers und/oder Polyäthylenglycoldimethyläthers als Katalysator in Abwesenheit eines Lösungsmittels auf nicht mehr als 200°C erhitzt.

Bei dem erfindungsgemäßen Verfahren ist es besonders wichtig, daß während der gesamten Reaktionszeit eine gute Durchmischung der Reaktionssuspension gewährleistet ist.

Die Reaktionstemperatur kann sich innerhalb eines Bereichs von etwa 125 bis etwa 200°C bewegen; zweckmäßigerweise wählt man die Temperaturen jedoch zwischen etwa 140 und etwa 190°C, weil dann einerseits eine genügende Reaktionsgeschwindigkeit herrscht und andererseits der Katalysator relativ stabil bleibt.

Als Katalysatoren können quartäre Ammoniumverbindungen, insbesondere Tetraalkyl(C₁ bis C₂₂)-ammoniumhalogenide, Tetraarylammoniumhalogenide, wobei die Arylreste beispielsweise Phenyl- oder Naphthylreste sein können, die durch Halogenatome, verzweigte oder unverzweigte Alkyl-, Nitro-, Cyano-, Amino- und/oder Alkoxygruppen substituiert sein können, und gemischte Alkylarylammoniumhalogenide, wie Octadecyltrimethylammoniumchlorid, Distearyldimethylammoniumchlorid, Tetramethylammoniumchlorid, Tetramethylammoniumbromid, Hexadecyltrimethylammoniumchlorid; Benzyltrimethylammoniumbromid; quartäre Phosphoniumverbindungen, insbesondere Tetraalkyl(C₁ bis C₂₂)-phosphoniumhalogenide, Tetraarylphosphoniumhalogenide, wobei die Arylreste beispielsweise Phenyl- oder Naphthylreste sein können, die durch Halogenatome, verzweigte oder unverzweigte Alkyl-, Nitro-, Cyano-, Amino- und/oder Alkoxygruppen substituiert sein können, und gemischte Alkylarylphosphoniumhalogenide wie Stearyltributylphosphoniumbromid, Hexadecyltriäthylphosphoniumbromid; Kronenäther, wie 18-Krone-6, Polyäthylenglycoldimethyläther und Kombinationen von diesen in katalytischen Mengen verwendet werden. Der Katalysator wurde bei dem erfindungsgemäßen Verfahren in Mengen von etwa 2 bis etwa 10 Gew.-%, vorzugsweise von etwa 3 bis etwa 5 Gew.-%, bezogen auf Dichlornitrobenzol, eingesetzt.

Als Alkalimetallfluoride können Lithiumfluorid, Natriumfluorid, Kaliumfluorid, Rubidiumfluorid, Cäsiumfluorid oder Kombinationen aus diesen eingesetzt werden, wobei Kaliumfluorid und Cäsiumfluorid bevorzugt sind.

Geeignete Dichlornitrobenzole sind beispielsweise 3,4-Dichlornitrobenzol, 2,5-Dichlornitrobenzol, 2,3-Dichlornitrobenzol und 2,4-Dichlornitrobenzol, die sich in die entsprechenden Chlorfluor- oder Difluornitrobenzole überführen lassen.

Was das Mengenverhältnis von Dichlornitrobenzol zu Alkalimetallfluorid angelangt, so werden zweckmäßigerweise etwa 1,05 bis etwa 1,7 mol Dichlornitrobenzol mit 1 mol Alkalimetallfluorid umgesetzt. Das Dichlornitrobenzol kann aber auch in einem molaren Überschuß bis 5:1 zur Anwendung gelangen. Durch die Anwendung des Dichlornitrobenzols im Überschuß kann die Ausbeute an Chlorfluor- bzw. Difluornitrobenzol wesentlich gesteigert werden. Außerdem wird dadurch sichergestellt, daß die Suspension nicht zu dickflüssig wird und damit noch rührfähig bleibt.

Das erfindungsgemäße Verfahren wird üblicherweise bei Atmosphärendruck ausgeführt. Es ist jedoch auch möglich bei Überdruck, beispielsweise bei bis zu 25 atm, zu arbeiten.

Von besonderem Vorteil bei dem erfindungsgemäßen Verfahren ist, daß weder die eingesetzten Dichlornitrobenzole und Alkalimetallfluoride noch der angewandte Katalysator zusätzlich getrocknet werden müssen, und daß auf den Einsatz eines Lösungsmittels verzichtet werden kann.

Die nachstehenden Beispiele dienen zur Erläuterung der Erfindung, ohne sie darauf zu beschränken.

### Beispiele

1. In einer Schmelze aus 2020 g (10,5 mol) 3,4-Dichlornitrobenzol wurden unter Rühren bei 50°C 70 g (0,2 mol) Octadecyltrimethylammoniumchlorid gelöst, bei 100°C 580 g (10,0 mol) Kaliumfluorid suspendiert und diese Suspension 9 h auf 180°C erwärmt. Nach Abkühlen auf ca. 60°C wurde die Reaktionssuspension abgesaugt, die am Rückstand haftende Flüssigkeit bei 150°C/70 torr abdestilliert und die vereinigten organischen Phasen unter Zusatz einer Hilfsbase als Säurefänger fraktioniert. Man erhielt so 1445 g (90,6 % bezogen auf umgesetztes 3,4-Dichlornitrobenzol) 3-Chlor-4-fluornitrobenzol und 324 g (13,6 % bezogen auf eingesetztes 3-4-Dichlornitrobenzol) nicht umgesetztes 3,4-Dichlornitrobenzol.
2. Analog Beispiel 1 wurden 2020 g (10,5 mol) 2,5-Dichlornitrobenzol, 70 g (0,2 mol) Octadecyltrimethylammoniumchlorid und 580 g (10,0 mol) Kaliumfluorid umgesetzt. Man erhielt so 936 g (72,3 % bezogen auf umgesetztes 2,5-Dichlornitrobenzol) 2-Fluor-5-chlornitrobenzol und 600 g (29,7 % bezogen auf eingesetztes 2,5-Dichlornitrobenzol) nicht umgesetztes 2,5-Dichlornitrobenzol.
3. Analog Beispiel 1 wurden 576 g (3 mol) 3,4-Dichlornitrobenzol, 58 g (0,1 mol) Distearyldimethylammoniumchlorid und 139 g (2,4 mol) Kaliumfluorid umgesetzt. Nach dem Absaugen wurde der Rückstand in Wasser gelöst, die organische Phase abgetrennt und die vereinigten organischen Phasen fraktioniert. Man erhielt 289 g (69 % bezogen auf eingesetztes Kaliumfluorid) 3-Chlor-4-fluornitrobenzol.
4. 96 g (0,5 mol) 3,4-Dichlornitrobenzol wurden mit 68 g (0,45 mol) Cäsiumfluorid und 8 g Distearyldimethylammoniumchlorid bei 180°C umgesetzt. Nach 4 h betrug der Umsatz über 70 % (GC) unter Erhalt von 3-Chlor-4-fluornitrobenzol.
5. 96 g (0,5 mol) 3,4-Dichlornitrobenzol wurden mit 26 g (0,45 mol) Kaliumfluorid und 4 g Distearyldimethylammoniumchlorid und 4 g Tetraethylenglycoldimethylether bei 180°C umgesetzt. Nach 4 h betrug der Umsatz über 50 % (GC) unter Erhalt von 3-Chlor-4-fluornitrobenzol.
6. 101 g (0,53 mol) 2,3-Dichlornitrobenzol wurden mit 29 g (0,5 mol) Kaliumfluorid und 5 g Tetrabutylphosphoniumbromid bei 180°C umgesetzt. Nach 19 h betrug der Umsatz über 65 % (GC) unter Erhalt von 2-Fluor-3-chlornitrobenzol.
7. 96 g (0,5 mol) 3,4-Dichlornitrobenzol wurden mit 26 g (0,45 mol) Kaliumfluorid und 7 g Polyäthylenglycoldimethyläther 1000 bei 200°C umgesetzt. Nach 19 h betrug der Umsatz 37 % (GC) unter Erhalt von 3-Chlor-4-fluornitrobenzol.
8. 96 g (0,5 mol) 3,4-Dichlornitrobenzol wurden mit 26 g (0,45 mol) Kaliumfluorid und 1,7 g 18-Krone-6 bei 180°C umgesetzt. Nach 21 h betrug der Umsatz 83 % (GC) unter Erhalt von 3-Chlor-4-fluornitrobenzol.
9. 96 g (0,5 mol) 3,4-Dichlornitrobenzol wurden mit 23 g (0,4 mol) Kaliumfluorid und 4 g Stearyltributylphosphoniumbromid und 0,8 g 18-Krone-6 bei 180°C umgesetzt. Nach 16 h betrug der Umsatz 72 % (GC) unter Erhalt von 3-Chlor-4-fluornitrobenzol.
10. 96 g (0,5 mol) 3,4-Dichlornitrobenzol wurden mit 28 g (0,45 mol) Kaliumfluorid/Cäsiumfluorid im Verhältnis 9:1 und 8 g Benzyltrimethylammoniumbromid bei 180°C umgesetzt. Nach 21 h war der Umsatz über 70 % (GC) unter Erhalt von 3-Chlor-4-fluornitrobenzol.
11. 202 g (1,1 mol) 2,3-Dichlornitrobenzol wurden mit 56 g (1,0 mol) Kaliumfluorid und 10 g Tetramethylammoniumchlorid bei 180°C umgesetzt. Nach 19 h war der Umsatz über 70 % (GC) unter Erhalt von 3-Chlor-2-fluornitrobenzol.
12. 202 g (1,1 mol) 2,3-Dichlornitrobenzol wurden mit 56 g (1,0 mol) Kaliumfluorid und 10 g Tetramethylammoniumbromid bei 180°C umgesetzt. Nach 19 h war der Umsatz über 70 % (GC) unter Erhalt von 3-Chlor-2-fluor-nitrobenzol.
13. 101 g (0,53 mol) 2,4-Dichlornitrobenzol wurden mit 58 g (1,0 mol) Kaliumfluorid und 5 g Hexadecyltriethylphosphoniumbromid bei 180°C umgesetzt. Nach 21 h betrug der Umsatz über 65 % (GC) unter Erhalt von 2,4-Difluornitrobenzol.

## Patentansprüche

1. Verfahren zur Herstellung von Chlorfluornitrobenzolen und Difluornitrobenzolen, dadurch gekennzeichnet, daß man Dichlornitrobenzol im Überschuß mit einem Alkalimetallfluorid mit einem Gesamtwassergehalt von etwa 0,2 bis etwa 2,5 Gew.-% in Gegenwart eines quartären Ammonium- und/oder Phosphoniumsalzes, Kronenäthers und/oder Polyäthylenglycoldimethyläthers als Katalysator in Abwesenheit eines Lösungsmittels auf nicht mehr als 200°C erhitzt.

2. Verfahren nach Anspruch 1, dadurch gekennzeichnet, daß als quartäre Ammoniumsalze Tetraalkyl(C₁ bis C₂₂)-ammoniumhalogenide, Tetraarylammoniumhalogenide, wobei die Arylreste substituiert sein können, und/oder gemischte Alkylarylammoniumhalogenide, als quartäre Phosphoniumsalze Tetraalkyl(C₁ bis C₂₂)-phosphoniumhalogenide, Tetraarylphosphoniumhalogenide, wobei die Arylreste substituiert sein können, und/oder gemischte Alkylarylphosphoniumhalogenide und als Kronenäther 18-Krone-6 eingesetzt werden.

3. Verfahren nach Anspruch 1, dadurch gekennzeichnet, daß als Katalysator Octadecyltrimethylammoniumchlorid, Distearyldimethylammoniumchlorid, Tetramethylammoniumchlorid, Tetramethylammoniumbromid, Hexadecyltrimethylammoniumchlorid, Benzyltrimethylammoniumbromid, Stearyltributylphosphoniumbromid und/oder Hexadecyltriäthylphosphoniumbromid eingesetzt werden.

4. Verfahren nach einem oder mehreren der Ansprüche 1 bis 3, dadurch gekennzeichnet, daß man den Katalysator in Mengen von etwa 2 bis etwa 10 Gew.-%, bezogen auf die eingesetzte Menge Dichlornitrobenzol, einsetzt.

5. Verfahren nach einem oder mehreren der Ansprüche 1 bis 3, dadurch gekennzeichnet, daß man den Katalysator in Mengen von etwa 3 bis etwa 5 Gew.-%, bezogen auf die eingesetzte Menge Dichlornitrobenzol, einsetzt.

6. Verfahren nach einem oder mehreren der Ansprüche 1 bis 5, dadurch gekennzeichnet, daß man etwa 1,05 bis etwa 1,7 mol Dichlornitrobenzol mit 1 mol Alkalimetallfluorid umsetzt.

7. Verfahren nach einem oder mehreren der Ansprüche 1 bis 6, dadurch gekennzeichnet, daß man als Alkalimetallfluorid Kaliumfluorid und/oder Cäsiumfluorid einsetzt.

8. Verfahren nach einem oder mehreren der Ansprüche 1 bis 7, dadurch gekennzeichnet, daß man die Dichlornitrobenzole, Alkalimetallfluoride und Katalysatoren in handelsüblicher Form, ohne zusätzliche Trocknung verwendet.

9. Verfahren nach einem oder mehreren der Ansprüche 1 bis 8, dadurch gekennzeichnet, daß die Reaktionstemperatur etwa 125 bis etwa 200°C beträgt.

10. Verfahren nach einem oder mehreren der Ansprüche 1 bis 8, dadurch gekennzeichnet, daß die Reaktionstemperatur etwa 140 bis etwa 190°C beträgt.

11. Verfahren nach einem oder mehreren der Ansprüche 1 bis 10, dadurch gekennzeichnet, daß bei Atmosphärendruck oder bei Überdruck gearbeitet wird.

## Claims

1. A process for the preparation of chlorofluoronitrobenzenes and difluoronitrobenzenes, which comprises heating dichloronitrobenzene to not more than 200°C in excess with an alkali metal fluoride having a total water content of about 0.2 to about 2.5% by weight in the presence of a quaternary ammonium and/or phosphonium salt, crown ether and/or polyethylene glycol dimethyl ether as catalyst in the absence of a solvent.

2. The process as claimed in claim 1, wherein the quaternary ammonium salts employed are tetraalkyl(C₁ to C₂₂)-ammonium halides, tetraarylammonium halides, it being possible for the aryl radicals to be substituted, and/or mixed alkylarylammonium halides, the quaternary phosphonium salts employed are tetraalkyl(C₁ to C₂₂)-phosphonium halides, tetraarylphosphonium halides, it being possible for the aryl radicals to be substituted, and/or mixed alkylarylphosphonium halides, and the crown ether employed is 18-crown-6.

3. The process as claimed in claim 1, wherein the catalyst employed is octadecyltrimethylammonium chloride, distearyldimethylammonium chloride, tetramethylammonium chloride, tetramethylammonium bromide, hexadecyltrimethylammonium chloride, benzyltrimethylammonium bromide, stearyltributylphosphonium bromide and/or hexadecyltriethylphosphonium bromide.

4. The process as claimed in one or more of claims 1 to 3, wherein the catalyst is employed in amounts of about 2 to about 10% by weight, relative to the amount of dichloronitrobenzene employed.

5. The process as claimed in one or more of claims 1 to 3, wherein the catalyst is employed in amounts of about 3 to about 5% by weight, relative to the amount of dichloronitrobenzene employed.

6. The process as claimed in one or more of claims 1 to 5, wherein about 1.05 to about 1.7 mol of dichloronitrobenzene are reacted with 1 mol of alkali metal fluoride.

7. The process as claimed in one or more of claims 1 to 6, wherein the alkali metal fluoride employed is potassium fluoride and/or cesium fluoride.

8. The process as claimed in one or more of claims 1 to 7, wherein the dichloronitrobenzenes, alkali metal fluorides and catalysts are used in commercial form, without additional drying.

9. The process as claimed in one or more of claims 1 to 8, wherein the reaction temperature is about 125 to about 200°C.

10. The process as claimed in one or more of claims 1 to 8, wherein the reaction temperature is about 140 to about 190°C.

11. The process as claimed in one or more of claims 1 to 10, which process is carried out at atmospheric pressure or at elevated pressure.

## Revendications

1. Procédé de préparation de chlorofluoronitrobenzènes et de difluoronitrobenzènes, caractérisé en ce qu'on chauffe à une température ne plus que 200°C un dichloronitrobenzène en excès avec un fluorure de métal alcalin ayant une teneur totale en eau comprise entre environ 0,2 et environ 2,5 % en poids, en présence d'un sel d'ammonium quaternaire et/ou de phosphonium, d'un éther couronne et/ou d'un poly-éthylèneglycoldiméthyléther en tant que catalyseur, en l'absence d'un solvant.

2. Procédé selon la revendication 1, caractérisé en ce qu'on utilise en tant que sels d'ammonium quaternaire des halogénures de tétra-(alkyl en C₁-C₂₂)-ammonium, des halogénures de tétraarylammonium, les restes aryles pouvant être substitués, et/ou des halogénures d'alkylarylammonium mixtes, en tant que sels de phosphonium quaternaire des halogénures de tétra-(alkyl en C₁-C₂₂)-phosphonium, des halogénures de tétraarylphosphonium, les restes aryles pouvant être substitués, et/ou des halogénures d'alkylarylphosphonium mixtes et en tant qu'éther couronne le 18-couronne-6.

3. Procédé selon la revendication 1, caractérisé en ce qu'on utilise en tant que catalyseur le chlorure d'octadécyltriméthylammonium, le chlorure de distéaryldiméthylammonium, le chlorure de tétraméthylammonium, le bromure de tétraméthylammonium, le chlorure d'hexadécyltriméthylammonium, le bromure de benzyltriméthylammonium, le bromure de stéaryltributylphosphonium et/ou le bromure d'hexadécyltriéthylphosphonium.

4. Procédé selon une ou plusieurs des revendications 1 à 3, caractérisé en ce qu'on utilise le catalyseur dans des quantités comprises entre environ 2 et environ 10 % en poids, par rapport à la quantité utilisée de dichloronitrobenzène.

5. Procédé selon une ou plusieurs des revendications 1 à 3, caractérisé en ce qu'on utilise le catalyseur dans des quantités comprises entre environ 3 et environ 5 % en poids, par rapport à la quantité utilisée de dichloronitrobenzène.

6. Procédé selon une ou plusieurs des revendications 1 à 5, caractérisé en ce qu'on fait réagir entre environ 1,05 et environ 1,7 moles de dichloronitrobenzène avec 1 mole de fluorure de métal alcalin.

7. Procédé selon une ou plusieurs des revendications 1 à 6, caractérisé en ce qu'on utilise comme fluorure de métal alcalin le fluorure de potassium et/ou le fluorure de césium.

8. Procédé selon une ou plusieurs des revendications 1 à 7, caractérisé en ce qu'on utilise les dichloronitrobenzènes, les fluorures de métaux alcalins et les catalyseurs sous leur forme commerciale, sans séchage supplémentaire.

9. Procédé selon une ou plusieurs des revendications 1 à 8, caractérisé en ce que la température de la réaction est comprise entre environ 125 et environ 200°C.

10. Procédé selon une ou plusieurs des revendications 1 à 8, caractérisé en ce que la température de réaction est comprise entre environ 140 et environ 190°C.

11. Procédé selon une ou plusieurs des revendications 1 à 10, caractérisé en ce qu'on travaille à pression atmosphérique ou en surpression.
